# FASCICULE DE BREVET EUROPEEN

(11) **EP 1 242 439 B1**
(45) Date de publication et mention de la délivrance du brevet: **21.05.2003**
(21) Numéro de dépôt: 00990066.3
(22) Date de dépôt: 19.12.2000
(51) Int. Cl.: C07H 17/08

(54) **PROCEDE POUR PREPARER ET ISOLER LA 9-DEOXO-9(Z)-HYDROXYIMINOERYTHROMYCINE A**
VERFAHREN ZUR HERSTELLUNG UND ISOLIERUNG VON 9-DEOXO-9(Z)-HYDROXYIMINOERYTHROMYCIN A
METHOD FOR PREPARING AND ISOLATING 9-DEOXO-9(Z)-HYDROXYIMINOERYTHROMYCIN A

(30) Priorité: 20.12.1999 FR 9916106
(43) Date de publication de la demande: 25.09.2002
(73) Titulaire: MERIAL, 69002 Lyon (FR)
(72) Inventeur: BASSET, François, F-69100 Villeurbanne (US); DURAND, Thierry, 69410 Champagne au Mont d Or (FR); GUEVEL, Ronan, F-69008 Lyon (FR); LEON, Patrick, décédé (FR); LHERMITTE, Frédéric, F-69360 Saint Symphorien D'Ozon (FR); ODDON, Gilles, F-69003 Lyon (FR); PAUZE, Denis, F-69360 Solaize (FR)
(74) Mandataire: Kling, Simone
(86) Numéro de dépôt international: FR0003595
(87) Numéro de publication internationale: WO01046211

(56) Documents cités:
- EP-A- 0 503 932
- EP-A- 0 503 949
- EP-A- 0 508 699
- WO-A-98/31699
- WO-A-99/02541
- US-A- 5 808 017

## Description

La présente invention concerne un procédé pour préparer et isoler la 9-déoxo-9(Z)-hydroxyiminoérythromycine A (appelée ci-après 9(Z)-érythromycine-oxime ou 9(Z)-oxime) à partir de l'isomère E correspondant.

La présente invention se situe dans le domaine des antibiotiques macrolides de type érythromycine et concerne plus particulièrement leurs dérivés aza-macrolides qui font l'objet de la demande de brevet EP 508 699 et répondent à la formule générale suivante: 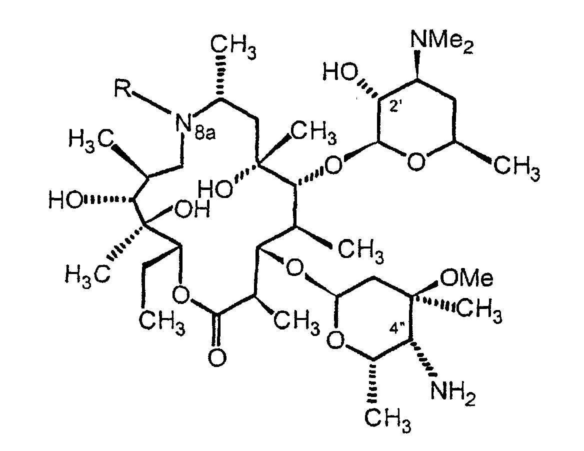 dans laquelle R représente un atome d'hydrogène, un groupement alkyle en C₁-C₁₀, alcényle en C₂-C₁₀ ou arylsulfonyle en C₆-C₁₂, le cas échéant substitués.

Ces composés sont obtenus à partir de l'érythromycine et leur synthèse implique deux étapes majeures :
- la création du macrocycle 8a-azalide au départ de la 9(E)-érythromycine-oxime isomérisée en 9(Z)-oxime correspondant, qui subit ensuite un réarrangement stéréospécifique de Beckmann, et
- la modification du groupe "cladinose" en position 4 consistant en la transformation du 4(S)-OH en 4(R)-NH₂.

La présente invention concerne plus particulièrement la première étape de cette synthèse et a pour objectif un nouveau procédé pour l'isomérisation de la 9(E)-érythromycine-oxime et l'isolation de l'isomère 9(Z)-oxime résultant que l'on peut illustrer comme suit :

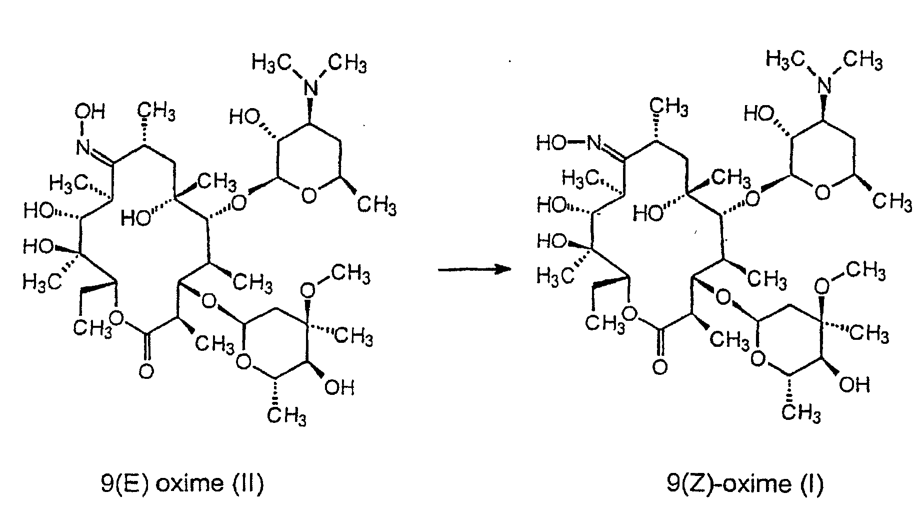

Cette étape d'isomérisation a fait l'objet en particulier de la demande de brevet EP 503 949 selon laquelle la 9(Z)-oxime de formule (I) est obtenue en traitant l'isomère (E) de formule (II) avec une base, de préférence un hydroxyde de métal alcalin tel que l'hydroxyde de lithium, dans un solvant protique ou aprotique qui est préférentiellement l'éthanol. Le résidu obtenu après évaporation du solvant est repris avec de l'acétate d'éthyle et une solution aqueuse qui est ensuite re-extraite avec de l'acétate d'éthyle pour conduire à un produit brut contenant un mélange d'oximes. Le mélange d'oximes brut est ensuite repris dans le chlorure de méthylène puis filtré. Le solide obtenu est alors repris dans l'acétate d'éthyle et un non solvant (nitrométhane) puis cristallisé ou purifié dans l'acétate d'éthyle par des étapes successives de précipitations avec du chlorure de méthylène, et filtrations.

En fait, les conditions actuelles ne sont pas extrapolables à l'échelle industrielle.

Ce procédé implique en effet, des concentrations à sec de la masse réactionnelle dans l'éthanol et de celle dans l'acétate d'éthyle.

Il implique également l'utilisation de solvants chlorés indésirables du point de vue de la protection de l'environnement.

Enfin, le produit isolé contient encore de l'isomère (E) et plusieurs reprises dans un milieu contenant de l'acétate d'éthyle et du chlorure de méthylène sont nécessaires afin de cristalliser (par "battage") l'isomère (Z) désiré et de l'isoler avec une pureté isomérique acceptable.

L'objectif de la présente invention est de fournir une alternative efficace au procédé connu permettant de remédier aux inconvénients précités.

L'invention a ainsi pour objectif de fournir un procédé simplifié, facile à mettre en oeuvre à l'échelle industrielle et permettant d'obtenir l'oxime-9(Z) avec une pureté isomérique satisfaisante.

L'objectif de l'invention est d'éviter notamment l'emploi de solvants chlorés néfastes du point de vue de l'environnement ainsi que la purification laborieuse par « battage » dans un mélange acétate d'éthyle/chlorure de méthylène.

La présente invention a pour objet un procédé pour la préparation de la 9-déoxo-9(Z)-hydroxyiminoérythromycine A répondant à la formule (I) suivante : 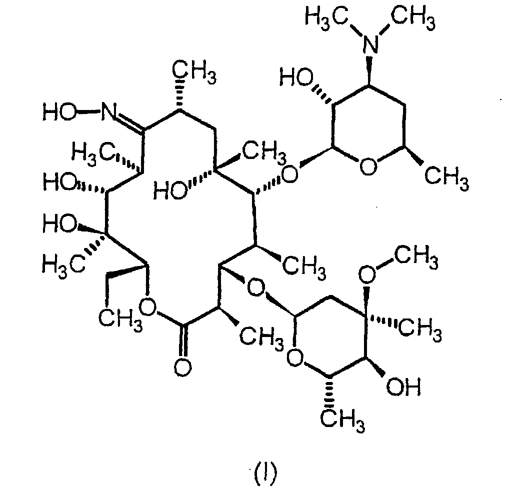 comprenant successivement les étapes consistant à :
- faire réagir dans l'eau, la 9-déoxo-9(E)-hydroxyiminoérythromycine A répondant à la formule (II) suivante : 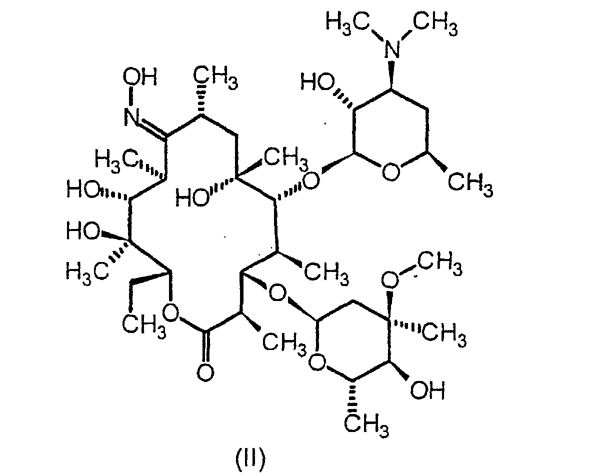
avec une base,
- acidifier le mélange réactionnel jusqu'à un pH compris entre 9 et 11 ;
- ajouter audit mélange un solvant organique ;
- éventuellement concentrer sous vide la phase organique résultante ;
- isoler la 9(Z)-érythromycine-oxime désirée.

Selon une variante préférée de l'invention, pour la réaction de la 9(E)-oxime de formule (II), on ajoute à l'eau un solvant organique de type dialkylcétone, en particulier l'acétone.

Les inventeurs ont mis en évidence, de manière inattendue, que l'on pouvait isomériser avec une base la 9-(E)-érythromycine-oxime en suspension dans l'eau éventuellement additionnée d'un solvant de type dialkylcétone, sans la présence d'un solvant alcoolique, puis extraire directement après neutralisation du sel, l'isomère désiré à partir de la suspension réactionnelle et l'isoler avec une pureté satisfaisante.

Ils ont ainsi mis en évidence que l'on pouvait obtenir l'isomère (Z) désiré par addition à la suspension réactionnelle, d'un solvant organique tel que l'acétate d'éthyle permettant d'induire sa cristallisation, sans ajout d'un autre solvant dans le milieu. Selon les cas, celui-ci peut être capable de former un solvat insoluble ou peu soluble avec la 9(Z)-oxime. Il ne nécessite pas de cristallisations subséquentes.

Selon un mode de réalisation, l'invention s'étend ainsi à l'utilisation, après isomérisation dans l'eau, de tout solvant organique capable d'induire la cristallisation de la 9(Z)-oxime, notamment par concentration, dans ledit solvant, alors que l'isomère (E) reste majoritairement en solution dans le milieu.

Selon une variante préférée, c'est l'isomérisation proprement dite qui est réalisée dans l'eau additionnée d'un solvant organique de type dialkylcétone capable de former un solvat cristallisable avec l'isomère (Z) comme indiqué ci-dessus.

Les inventeurs ont ainsi mis au point un procédé simplifié permettant de s'affranchir de la précipitation au chlorure de méthylène ainsi que de la purification multi-étapes nécessaire dans le procédé connu.

Les inventeurs ont également mis en évidence que l'on pouvait avantageusement recycler les jus-mères (contenant un mélange des isomères E et Z) recueillis après isolation de l'isomère (Z) cristallisé, en reformant une suspension aqueuse du mélange d'isomères qu'ils contiennent après élimination de la majeure partie du solvant organique présent.

Le procédé selon l'invention va être décrit plus en détails ci-après :
Celui-ci consiste tout d'abord à traiter la 9(E)-érythromycineoxime, en suspension dans l'eau, par une base de préférence hydrosoluble.

Selon une variante préférée de l'invention, la 9(E)-oxime est mise en réaction avec la base, dans un milieux aqueux formé d'eau en mélange avec un solvant organique- de type dialkylcétone et avantageusement capable de former un solvat cristallisable avec la 9(Z)-oxime alors que l'isomère (E) reste majoritairement en solution. Le solvant de type dialkylcétone est préférentiellement choisi parmi les dialkylcétones contenant de 3 à 10 atomes de carbone et il s'agit typiquement d'acétone.

On ajoute ensuite la base pour donner lieu à la réaction d'isomérisation.

On peut citer à titre d'exemples de base, les hydroxydes de métal alcalin ou alcalino-terreux, les ammoniums, les carbonates, les alcoxydes. Celle-ci consiste préférentiellement en de la lithine ou de la soude.

La base est utilisée en une quantité de préférence comprise entre 1 et 10 équivalents, de préférence 2 équivalents molaires par rapport à la 9(E)-oxime.

L'addition de la base à la 9(E)-oxime conduit à la déprotonation de celle-ci et permet d'atteindre les conditions d'équilibre avec l'isomère (Z).

Le pH du milieu réactionnel est généralement compris entre 11,5 et 14.

Le traitement subséquent appliqué au mélange permet de déplacer cet équilibre et d'isoler préférentiellement la 9(Z)-oxime sous forme de solvat.

La réaction est généralement conduite sous atmosphère inerte. Le rapport Z/E est température-dépendant et on préfère effectuer la réaction à une température comprise entre 10° et 25°C, plus préférentiellement voisine de 20°C.

Le milieu réactionnel est de préférence agité pendant 6 à 24 heures.

L'isomère (Z) désiré est ensuite extrait avec un solvant organique, en particulier avec de l'acétate d'éthyle ou autre solvant équivalent.

Pour ce faire, le mélange réactionnel est tout d'abord acidifiée jusqu'à un pH de préférence compris entre 9 et 11, plus préférentiellement encore jusqu'à un pH de l'ordre de 9,5-10. Pour cela, on utilise de préférence de l'acide chlorhydrique, de l'acide acétique ou du bicarbonate de sodium.

Pour réaliser cette étape d'acidification, ledit mélange est de préférence refroidi à une température inférieure à 20°C, et plus préférentiellement à une température de l'ordre de 10°C.

Un solvant organique est ensuite ajouté au milieu réactionnel afin d'induire la cristallisation de l'isomère (Z) recherché.

Lorsque la réaction d'isomérisation est conduite dans l'eau, selon un mode préféré de réalisation de l'invention, on utilise de l'acétate d'éthyle ou d'autres solvants présentant des propriétés équivalentes de cristallisation de l'isomère (Z).

Par "solvants présentant des propriétés équivalentes de cristallisation de l'isomère (Z)" on entend tout solvant capable d'induire la cristallisation de la 9(Z)-oxime, notamment par concentration de la phase organique d'extraction, alors que l'isomère (E) reste majoritairernent en solution.

On pense en effet que, selon ce mode de mise en oeuvre de l'invention, l'isomère (Z) peut être directement extrait du milieu réactionnel et peut cristalliser par concentration dans le solvant organique d'extraction. Dans le cas où la réaction d'isomérisation est conduite dans un mélange eau/dialkylcétone, c'est le solvat de la (Z)-oxime avec la dialkylcétone qui précipite en fin de neutralisation. L'utilisation d'un solvant organique tel que l'acétate d'éthyle ou le méthylbutyléther permet d'améliorer le ratio Z/E au profit de l'isomère (Z) recherché, lors de la filtration du solvat avec la dialkylcétone. D'autre part, un ester tel que l'acétate d'éthyle permet en plus d'améliorer le séchage ultérieur de l'oxime (Z) en favorisant l'élimination du solvant de type dialkylcétone.

Lors de l'étape d'extraction, la température du milieu réactionnel est de préférence ramenée à la température ambiante (de l'ordre de 25-30°C) ce qui facilite la décantation.

Après décantation, la phase aqueuse est de préférence à nouveau extraite dans les conditions précitées.

Le cas échéant, les phases organiques d'extraction sont combinées puis concentrées sous vide afin de provoquer la cristallisation de l'isomère (Z) recherché dans le milieu.

La température du milieu réactionnel est de préférence maintenue inférieure à 35°C lors de cette opération de concentration et est de préférence réalisée en plusieurs heures (de l'ordre de 4 à 5 heures).

L'isomère (Z) recherché est alors isolé par filtration. Pour cela, la masse réactionnelle est de préférence maintenue entre 10 et 25°C, de préférence refroidie jusqu'à une température de l'ordre de 10°C.

L'isomère (Z) est récupéré avec un ratio Z/E supérieur à 90/10, typiquement compris entre 93/7 et 98/2.

Selon l'invention, les jus-mères recueillis après filtration, qui contiennent essentiellement la 9(E)-oxime, peuvent être à nouveau traités comme indiqué précédemment.

Dans ce cas, la majeure partie de l'acétate d'éthyle ou autre solvant d'extraction est distillée sous vide sur un pied d'eau, jusqu'à ce qu'il n'en reste par exemple que 3 à 4 %.

On ajoute alors le cas échéant un solvant de type dialkylcétone et de la base comme indiqué ci-dessus, afin de réaliser une nouvelle isomérisation de la 9(E)-oxime présente puis l'isolation de la 9(Z)-oxime formée dans les conditions indiquées précédemment.

Selon le solvant d'extraction, on peut être amené à introduire une quantité plus importante de base en raison d'une éventuelle saponification du solvant.

Le procédé selon l'invention présente l'avantage de ne mettre en oeuvre qu'un seul solvant d'extraction, généralement non chloré, et de ne pas nécessiter de reprises multiples afin d'obtenir la cristallisation de l'isomère recherché. Il peut être facilement mis en oeuvre d'un point de vue industriel.

Le procédé selon l'invention est illustré ci-après par des exemples qui ne doivent pas être considérés comme limitatifs.

### EXEMPLE 1 : réaction d'isomérisation dans l'eau :

Dans un réacteur homothétique de 1 litre pour atmosphère d'azote, on charge la 9-(E)-érythromycin-oxime (II) (50 g, 0,065 mol, 1 équiv.), la lithine LiOH.H₂O (5,7 g, 0,133 mol) puis on ajoute de l'eau distillée (500 ml) en rinçant bien l'entonnoir qui a servi à ajouter les solides.

La suspension de 9-(E)-érythromycine-oxime ainsi obtenue est agitée pendant 9 heures ou plus à une température de 16°C environ (ou à température ambiante). La consigne de température est ensuite refroidie à 10°C environ et une solution d'HCl 1N est coulée en 1h30 ou plus de manière à amener le pH de la masse réactionnelle à une valeur de 9,5 environ.

La suspension ainsi obtenue est extraite par de l'acétate d'éthyle (300 g). Pour améliorer l'extraction, la masse réactionnelle est chauffée à 25-30°C environ. Après décantation, la phase aqueuse est contre-extraite par de l'acétate d'éthyle (2 x 225 g). Les phases organiques combinées sont ensuite concentrées sous vide par distillation partielle de l'acétate d'éthyle. La masse réactionnelle est ensuite refroidie à environ 10°C pendant 1h30 environ puis filtrée.

On isole après filtration et séchage 32 g de 9-(Z)-érythromycine-oxime (I) (ratio Z : E = 96 : 4 par RMN - ¹H). Les jus mères isolés (97 g) peuvent être recyclés comme décrit à l'exemple 2.

### EXEMPLE 2 : recyclage des jus mères :

Dans un réacteur homothétique de 1 litre sous atmosphère d'azote, on charge les jus-mères de filtration (97 g) puis on ajoute de l'eau distillée (500 ml). On distille sous vide l'acétate d'éthyle jusqu'à ce qu'il ne reste que 3 à 4% en poids d'acétate d'éthyle. La lithine LiOH.H₂O (4,7 g, 0,109 mol) est ensuite chargée.

La suspension du mélange d'érythromycine-oximes Z et E ainsi obtenue est agitée à environ 800 tpm pendant 10 heures ou plus à une température de 16°C environ (ou à température ambiante). La consigne de température est ensuite refroidie à 10°C environ et une solution d'HCI 1N est coulée en 1h30 ou plus de manière à amener le pH de la masse réactionnelle à une valeur de 9,5 environ. On ajoute ensuite l'acétate d'éthyle (300 g) puis on chauffe la masse à 25-30°C environ. Après décantation, la phase aqueuse est contre-extraite par de l'acétate d'éthyle (2 x 225 g). La phase organique est rapidement transférée dans le réacteur puis concentrée sous vide par distillation partielle de l'acétate d'éthyle jusqu'à un volume minimal agitable. La masse réactionnelle obtenue est ensuite refroidie à 10°C environ pendant 1h30 environ puis filtrée.

On isole après filtration et séchage 6 g de 9-(Z)-érythromycine-oxime (I) (ratio Z : E ≥ 96 : 4 par RMN - 1H).
Rendement pondéral = 76 %

### EXEMPLE 3 : réaction d'isomérisation dans un mélange eau/acétone:

Dans un réacteur de 1 litre inerté à l'azote, on charge la 9(E)-érythromycine-oxime A (115 g, 0, 150 mol, 1 équiv.) puis l'eau potable (220 g) et l'acétone (272 g) en rinçant bien l'entonnoir. La suspension ainsi obtenue est traitée par de la soude 30% (38 g ; 1,9 équiv.) puis agitée pendant 8 heures ou plus à température ambiante. La solution est ensuite neutralisée par l'addition d'acide acétique en 1 heure environ ou plus de manière à amener le pH de la masse réactionnelle à une valeur de 10 environ.

A ce stade, il s'est formé un solvat des érythromycine-oximes avec l'acétone (ratio molaire 1:1 par ¹H-RMN) qui précipite dans le milieu réactionnel.

La suspension ainsi obtenue est traitée par de l'acétate d'éthyle (200 g) puis agitée pendant 3 heures minimum à température ambiante puis refroidie à 0°C environ. Après 3 heures environ d'agitation à 0°C, la suspension est filtrée puis lavée par de l'eau potable (360 g).

Le produit obtenu est ensuite repris avec de l'acétate d'éthyle (173 g) à 40°C environ et agité à cette température pendant 3 heures environ. La suspension obtenue est refroidie à température ambiante puis filtrée.

On isole après filtration et séchage à 50°C 78 g de 9-(Z)-érythromycine-oxime (ratio Z : E ≥ 97:3 par HPLC).

Les données (distance et intensité relative) obtenues par l'analyse aux rayons X de la 9(Z)-oxime (I) sous forme de solvat avec l'acétone, sont rapportées ci-dessous :

| d(hkl) Å | Intensité(%) |
|---|---|
| 13,12 | 27 |
| 11,86 | 23 |
| 11,69 | 26 |
| 11,31 | 46 |
| 10,05 | 30 |
| 9,78 | 67 |
| 9,02 | 33 |
| 8,79 | 63 |
| 8,04 | 100 |
| 7,44 | 26 |
| 7,38 | 31 |
| 7,09 | 29 |
| 7,03 | 35 |
| 6,79 | 25 |
| 6,59 | 26 |
| 6,54 | 30 |
| 5,97 | 43 |
| 5,63 | 26 |
| 5,59 | 24 |
| 5,23 | 17 |
| 5,01 | 25 |
| 4,93 | 49 |
| 4,87 | 31 |
| 4,75 | 25 |
| 4,58 | 36 |
| 4,26 | 18 |
| 4,14 | 25 |
| 3,90 | 10 |
| 3,75 | 12 |
| 3,68 | 11 |
| 3,35 | 8 |
| 3,17 | 7 |
| 3,10 | 5 |
| 2,98 | 6 |
| 2,71 | 5 |
| 2,42 | 5 |

## Revendications

1. Procédé pour la préparation de la 9-déoxo-9(Z)-hvdroxviminoérythromycine A répondant à la formule (I) suivante : 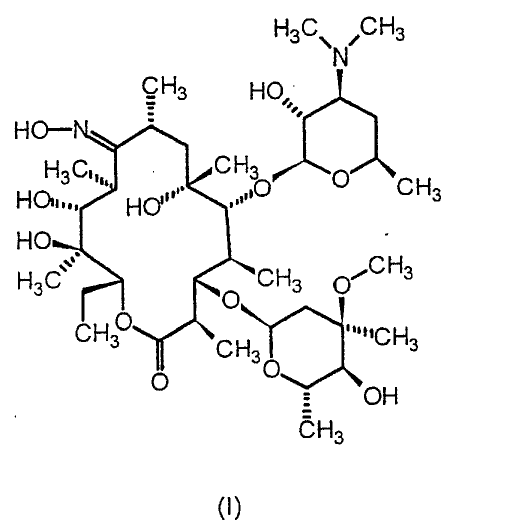 comprenant successivement les étapes consistant à :
- faire réagir dans l'eau la 9-déoxo-9(E)-hydroxyiminoérythromycine A répondant à la formule (II) suivante : 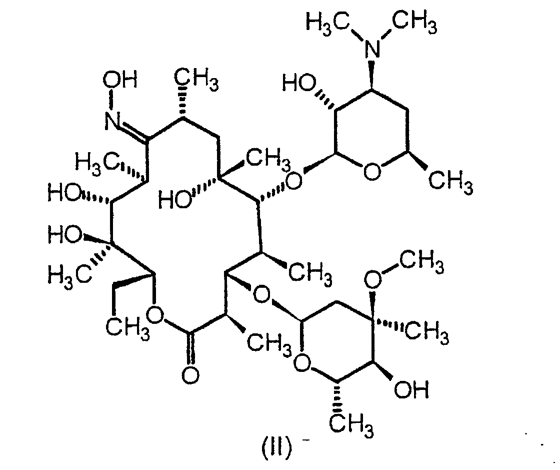
avec une base,
- acidifier le mélange réactionnel jusqu'à un pH compris entre 9 et 11 ;
- ajouter audit mélange un solvant organique ;
- éventuellement concentrer sous vide la phase organique résultante ;
- isoler la 9(Z)-érythromycine-oxime désirée.

2. Procédé selon la revendication 1, **caractérisé en ce que** pour faire réagir la 9(E)-oxime de formule (II) avec une base, on ajoute à l'eau un solvant organique de type dialkylcétone capable de former un solvat cristallisable avec la 9(Z)-oxime.

3. Procédé selon la revendication 2, **caractérisé en ce que** le solvant organique de type dialkylcétone est choisi parmi les dialkylcétones contenant 3 à 10 atomes de carbone et consiste de préférence en l'acétone.

4. Procédé selon l'une quelconque des revendications 1 à 3, **caractérisé en ce que** la base est de préférence hydrosoluble et est choisie parmi les hydroxydes de métaux alcalin ou alcalino-terreux, les ammoniums, les carbonates, les alcoxydes.

5. Procédé selon la revendication 4, **caractérisé en ce que** l'hydroxyde de métal alcalin consiste en de la lithine ou de la soude.

6. Procédé selon l'une quelconque des revendications 1 à 5, **caractérisé en ce qu'**on utilise 1 à 10 équivalents molaires de base, de préférence 2 équivalents molaires par rapport à la 9(E)-oxime.

7. Procédé selon l'une quelconque des revendications précédentes, **caractérisé en ce que** l'opération d'isomérisation est réalisée à température comprise entre 10 et 25°C, de préférence voisine de 20°C.

8. Procédé selon l'une quelconque des revendications précédentes, **caractérisé en ce que** le mélange réactionnel est acidifié jusqu'à un pH de l'ordre de 9,5-10.

9. Procédé selon l'une quelconque des revendications précédentes, **caractérisé en ce que** l'acidification est réalisée à l'aide d'acide chlorhydrique, d'acide acétique ou de bicarbonate de sodium.

10. Procédé selon l'une quelconque des revendications précédentes, **caractérisé en ce que** l'étape d'acidification est réalisée à une température inférieure à 20°C, de préférence à une température de l'ordre de 10°C.

11. Procédé selon l'une quelconque des revendications précédentes, **caractérisé en ce que** lorsque la réaction d'isomérisation est conduite dans l'eau, le solvant organique ajouté est de l'acétate d'éthyle ou un solvant présentant des propriétés équivalentes de solvatation de l'isomère (Z).

12. Procédé selon la revendication 11, **caractérisé en ce que** le solvant organique est l'acétate d'éthyle.

13. Procédé selon l'une-quelconque des revendications 1 à 11, **caractérisé en ce que** lorsque la réaction d'isomérisation est conduite dans un mélange eau/dialkylcétone, le solvant organique ajouté est de type ester.

14. Procédé selon la revendication 13, **caractérisé en ce que** le solvant organique est de l'acétate d'éthyle.

15. Procédé selon l'une quelconque des revendications précédentes, **caractérisé en ce que**, lors de l'étape de concentration sous vide de la phase organique, la température du milieu est maintenue inférieure à 35°C.

16. Procédé selon l'une quelconque des revendications précédentes, **caractérisé en ce que** les jus-mères recueillis après filtration sont traités par le procédé défini selon l'une quelconque des revendications précédentes, la majeure partie du solvant organique étant préalablement éliminée du milieu avant addition d'eau, éventuellement du solvant de type dialkylcétone et de la base.

17. Composé 9-déoxo-9(Z)-hydroxyiminoérythromycine A de formule (I) suivante : 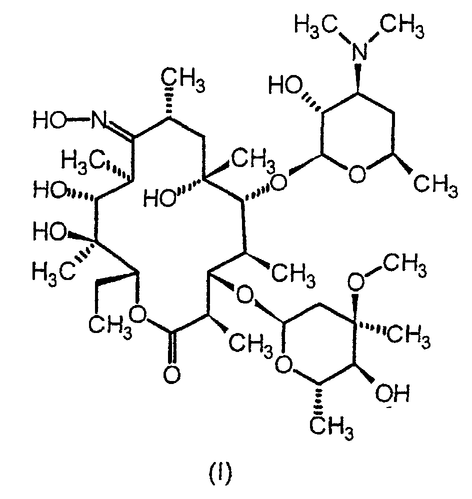 sous forme d'un solvat avec un solvant organique de type dialkylcétone.

18. Composé selon la revendication 17, sous forme d'un solvat avec une dialkylcétone contenant de 3 à 10 atomes de carbone.

19. Composé selon la revendication 18, sous forme d'un solvat avec l'acétone.

## Claims

1. Process for the preparation of 9-deoxo-9(Z)-hydroxyiminoerythromycin A corresponding to the following formula (I): 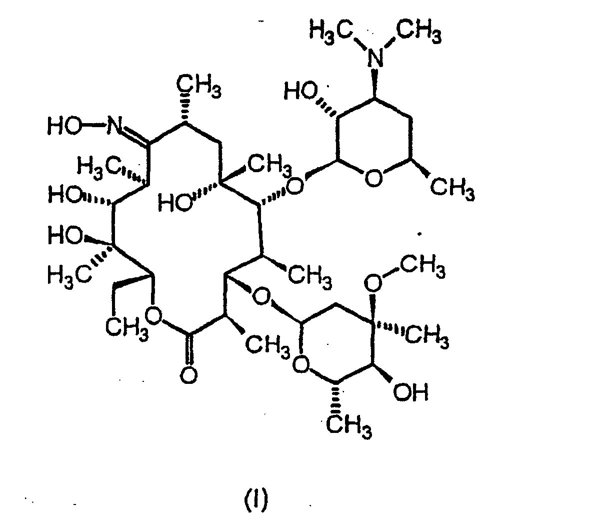 comprising, successively, the steps consisting of:
- reacting the 9-deoxo-9(E)-hydroxyiminoerythromycin A corresponding to the following formula (II) with a base, in water: 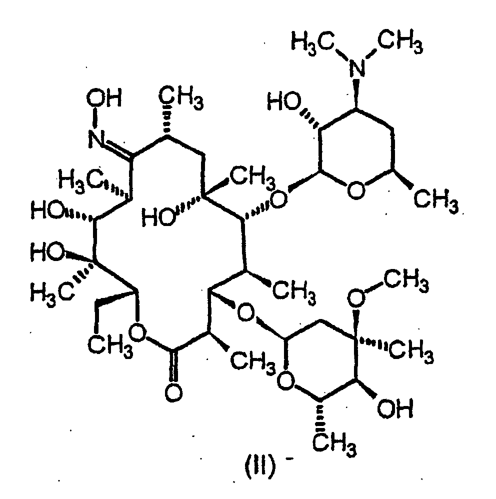
- acidifying the reaction mixture to a pH of between 9 and 11;
- adding an organic solvent to said mixture;
- optionally concentrating the resulting organic phase *in vacuo;*
- isolating the desired 9(Z)-erythromycin-oxime.

2. Process according to claim 1, **characterised in that**, in order to react the 9(E)-oxime of formula (II) with a base, an organic solvent of the dialkylketone type capable of forming a crystallisable solvate with the 9(Z)-oxime is added to the water.

3. Process according to claim 2, **characterised in that** the organic solvent of the dialkylketone type is selected from among the dialkylketones containing 3 to 10 carbon atoms and preferably consists of acetone.

4. Process according to any one of claims 1 to 3, **characterised in that** the base is preferably watersoluble and is selected from among the alkali metal or alkaline earth metal hydroxides, the ammoniums, carbonates and alkoxides.

5. Process according to claim 4, **characterised in that** the alkali metal hydroxide consists of lithium or sodium hydroxide.

6. Process according to any one of claims 1 to 5, **characterised in that** 1 to 10 molar equivalents, preferably 2 molar equivalents of base are used in relation to the 9(E)-oxime.

7. Process according to any one of the preceding claims, **characterised in that** the operation of isomerisation is carried out at a temperature between 10 and 25°C, preferably around 20°C.

8. Process according to any one of the preceding claims, **characterised in that** the reaction mixture is acidified to a pH of the order of 9.5 - 10.

9. Process according to any one of the preceding claims, **characterised in that** the acidification is carried out using hydrochloric acid, acetic acid or sodium bicarbonate.

10. Process according to any one of the preceding claims, **characterised in that** the acidification step is carried out at a temperature below 20°C, preferably at a temperature of the order of 10°C.

11. Process according to any one of the preceding claims, **characterised in that** when the isomerisation reaction is carried out in water, the organic solvent added is ethyl acetate or a solvent having equivalent solvating effects on the isomer (Z).

12. Process according to claim 11, **characterised in that** the organic solvent is ethyl acetate.

13. Process according to any one of claims 1 to 11, **characterised in that** when the isomerisation reaction is carried out in a mixture of water and dialkylketone, the organic solvent added is of the ester type.

14. Process according to claim 13, **characterised in that** the organic solvent is ethyl acetate.

15. Process according to any one of the preceding claims, **characterised in that** during the step of concentration of the organic phase *in vacuo* the temperature of the medium is kept below 35°C.

16. Process according to any one of the preceding claims, **characterised in that** the mother liquors collected after filtration are treated by the process as defined in any one of the preceding claims, the majority of the organic solvent having previously been eliminated from the medium before the addition of water, optionally the solvent of the dialkylketone type and the base.

17. The compound 9-deoxo-9(Z)-hydroxyiminoerythromycin A of the following formula (I): 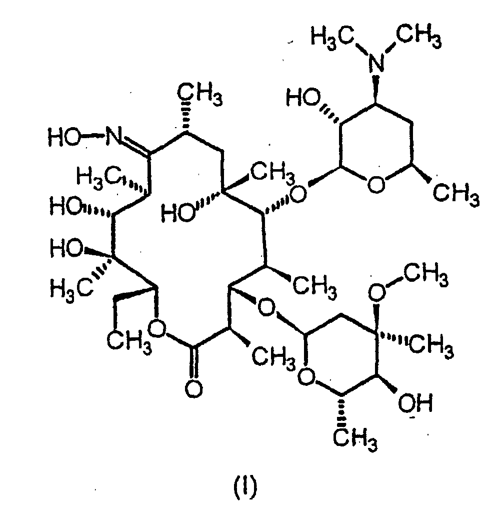 in the form of a solvate with an organic solvent of the dialkylketone type.

18. Compound according to claim 17 in the form of a solvate with a dialkylketone containing from 3 to 10 carbon atoms.

19. Compound according to claim 18 in the form of a solvate with acetone.

## Patentansprüche

1. Verfahren zur Herstellung von 9-Deoxo-9(Z)-hydroxyiminoerythromycin A mit der Formel (I) 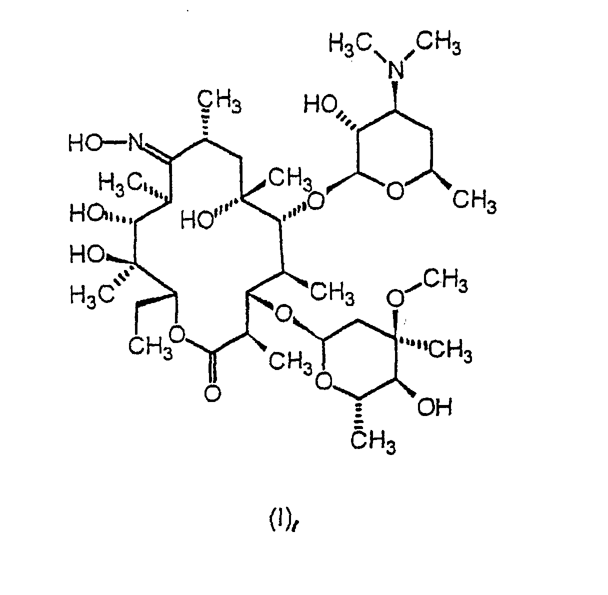 das nacheinander die Stufen umfasst, die darin bestehen
- 9-Deoxo-9(E)-hydroxyiminoerythromycin A mit der Formel (II) 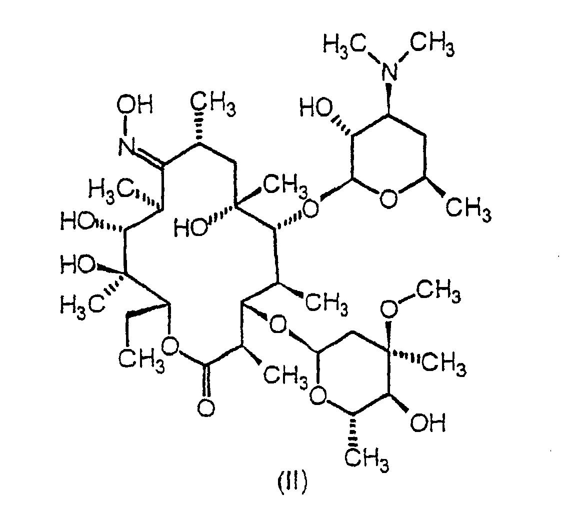
mit einer Base in Wasser umzusetzen,
- das Reaktionsgemisch bis auf einen pH-Wert von 9 bis 11 anzusäuern,
- ein organisches Lösungsmittel zu dem Gemisch zuzugeben,
- gegebenenfalls die resultierende organische Phase im Vakuum aufzukonzentrieren und
- das gewünschte 9(Z)-Erythromycinoxim zu isolieren.

2. Verfahren nach Anspruch 1, **dadurch gekennzeichnet, dass** für die Umsetzung des 9(E)-Oxims mit der Formel (II) mit einer Base ein organisches Lösungsmittel vom Typ Dialkylketon, das in der Lage ist, ein mit dem 9(Z)-Oxim auskristallisierbares Solvat zu bilden, zum Wasser zugegeben wird.

3. Verfahren nach Anspruch 2, **dadurch gekennzeichnet, dass** das organische Lösungsmittel vom Typ Dialkylketon aus 3 bis 10 Kohlenstoffatome enthaltenden Dialkylketonen ausgewählt wird und vorzugsweise aus Aceton besteht.

4. Verfahren nach einem der Ansprüche 1 bis 3, **dadurch gekennzeichnet, dass** die Base vorzugsweise wasserlöslich ist und aus Alkali- oder Erdalkalimetallhydroxiden, Ammoniumverbindungen, Carbonaten und Alkoxiden ausgewählt wird.

5. Verfahren nach Anspruch 4, **dadurch gekennzeichnet, dass** das Alkalimetallhydroxid aus Lithiumhydroxid oder Natriumhydroxid besteht.

6. Verfahren nach einem der Ansprüche 1 bis 5, **dadurch gekennzeichnet, dass** 1 bis 10 Moläquivalente und vorzugsweise 2 Moläquivalente Base auf das 9(E)-Oxim eingesetzt werden.

7. Verfahren nach einem der vorhergehenden Ansprüche, **dadurch gekennzeichnet, dass** die Isomerisierung bei einer Temperatur von 10 bis 25°C und vorzugsweise von etwa 20°C durchgeführt wird.

8. Verfahren nach einem der vorhergehenden Ansprüche, **dadurch gekennzeichnet, dass** das Reaktionsgemisch bis auf einen pH-Wert von etwa 9,5 bis 10 angesäuert wird.

9. Verfahren nach einem der vorhergehenden Ansprüche, **dadurch gekennzeichnet, dass** das Ansäuern mittels Salzsäure, Essigsäure oder Natriumhydrogencarbonat durchgeführt wird.

10. Verfahren nach einem der vorhergehenden Ansprüche, **dadurch gekennzeichnet, dass** die Ansäuerungsstufe bei einer Temperatur von unter 20°C und vorzugsweise bei einer Temperatur von etwa 10°C durchgeführt wird.

11. Verfahren nach einem der vorhergehenden Ansprüche, **dadurch gekennzeichnet, dass**, wenn die Isomerisierung in Wasser durchgeführt wird, das zugegebene organische Lösungsmittel Ethylacetat oder ein Lösungsmittel, das äquivalente Solvatisierungseigenschaften für das (Z)-Isomer besitzt, ist.

12. Verfahren nach Anspruch 11, **dadurch gekennzeichnet, dass** das organische Lösungsmittel Ethylacetat ist.

13. Verfahren nach einem der Ansprüche 1 bis 11, **dadurch gekennzeichnet, dass**, wenn die Isomerisierung in einem Wasser/Dialkylketon-Gemisch durchgeführt wird, das zugegebene organische Lösungsmittel ein Ester ist.

14. Verfahren nach Anspruch 13, **dadurch gekennzeichnet, dass** das organische Lösungsmittel Ethylacetat ist.

15. Verfahren nach einem der vorhergehenden Ansprüche, **dadurch gekennzeichnet, dass** in der Stufe der Aufkonzentrierung der organischen Phase im Vakuum die Temperatur des Mediums auf unter 35°C gehalten wird.

16. Verfahren nach einem der vorhergehenden Ansprüche, **dadurch gekennzeichnet, dass** die Mutterlaugen, die nach der Filtration erhalten werden, durch das Verfahren nach einem der vorhergehenden Ansprüche behandelt werden, wobei der größte Teil des organischen Lösungsmittels vor Zugabe des Wassers, gegebenenfalls des Lösungsmittels vom Typ Dialkylketon, und der Base entfernt worden ist.

17. 9-Deoxo-9(Z)-hydroxyiminoerythromicin-A-Verbindung mit der Formel (I) 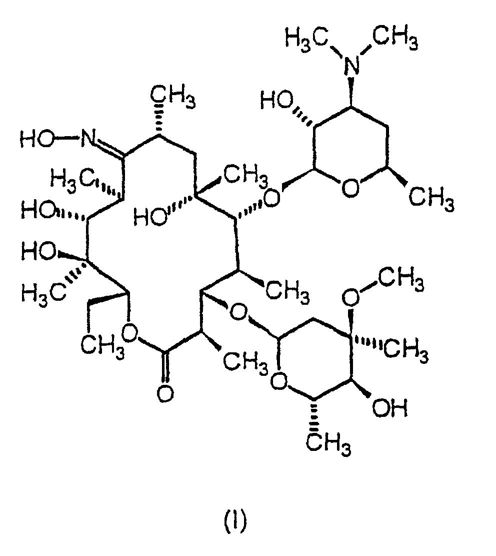 in Form eines Solvats mit einem organischen Lösungsmittel des Typs Dialkylketon.

18. Verbindung nach Anspruch 17 in Form eines Solvats mit einem 3 bis 10 Kohlenstoffatome enthaltenden Dialkylketon.

19. Verbindung nach Anspruch 18 in Form eines Solvats mit Aceton.
